(19) Europäisches Patentamt
European Patent Office
Office européen des brevets

(11) Veröffentlichungsnummer: **0 212 303 B1**

(12) **EUROPÄISCHE PATENTSCHRIFT**

(45) Veröffentlichungstag der Patentschrift: **12.06.91**

(51) Int. Cl.[5]: **C07B 43/04**, C07C 209/26, C07C 209/36, C07C 231/00, C07C 227/00

(21) Anmeldenummer: **86110103.8**

(22) Anmeldetag: **23.07.86**

(54) **Verfahren zur Herstellung aromatischer Dialkylamine.**

(30) Priorität: **07.08.85 DE 3528262**

(43) Veröffentlichungstag der Anmeldung:
**04.03.87 Patentblatt 87/10**

(45) Bekanntmachung des Hinweises auf die Patenterteilung:
**12.06.91 Patentblatt 91/24**

(84) Benannte Vertragsstaaten:
**BE CH DE FR GB IT LI NL SE**

(56) Entgegenhaltungen:
**EP-A- 0 014 998**
**DE-A- 2 120 641**
**US-A- 3 234 281**
**US-A- 3 522 309**
**US-A- 4 207 260**

**Journal of the American Chemical Society 63.1941.749**

(73) Patentinhaber: **HOECHST AKTIENGESELLSCHAFT**
**Postfach 80 03 20**
**W-6230 Frankfurt am Main 80(DE)**

(72) Erfinder: **Papenfuhs, Theodor, Dr.**
**Heinrich-Bleicher-Strasse 40**
**W-6000 Frankfurt am Main 50(DE)**
Erfinder: **Kühn, Walter, Dr.**
**Am Sonnenhang 7**
**W-6238 Hofheim am Taunus(DE)**

**EP 0 212 303 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein hinsichtlich Gewerbehygiene, Ökologie und Ausbeute verbessertes Verfahren zur Herstellung aromatischer Dialkylamine, die große Bedeutung als Kupplungskomponenten zur Herstellung von Dispersionsazofarbstoffen und zur Synthese von kationischen Farbstoffen haben.

Die technische Herstellung aromatischer Dialkylamine der allgemeinen Formel (1)

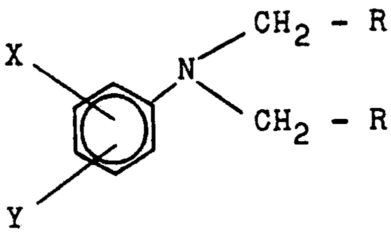

(1)

in welcher R eine Alkyl-$C_1$-$C_6$-Gruppe, X und Y je ein Wasserstoff, Fluor- oder Chloratom oder eine Hydroxy-, Alkyl-$C_1$-$C_4$-, Alkoxy-$C_1$-$C_4$-, Carboxy-, Carbalkoxy-$C_1$-$C_5$-,

Alkyl-$C_1$-$C_4$-CO-NH-,

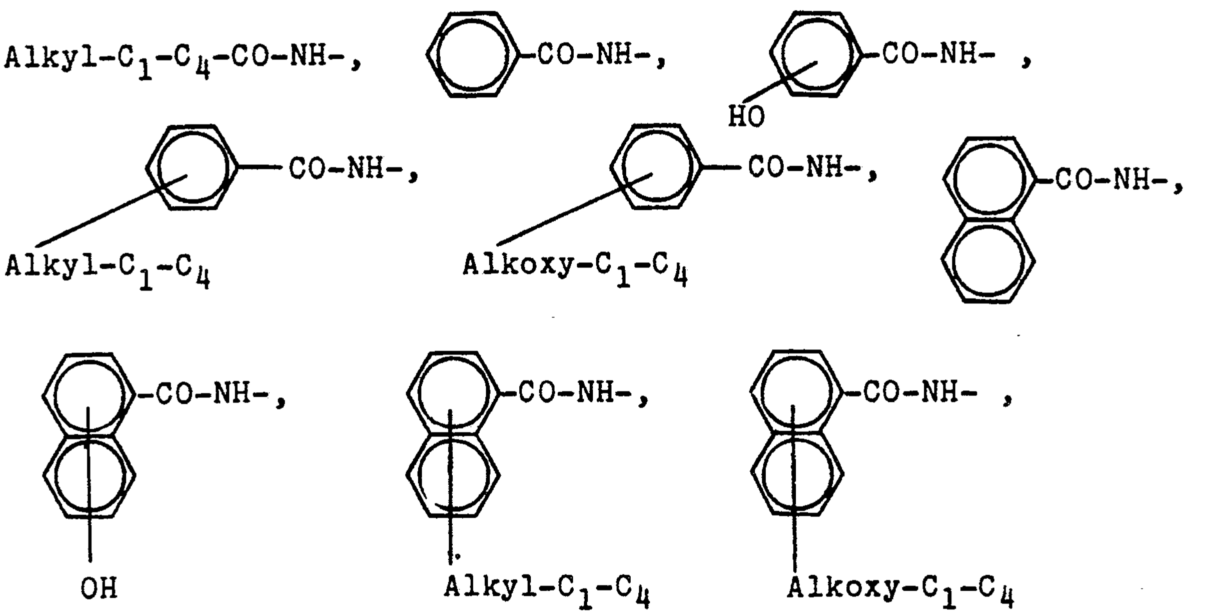

Sulfonsäure-, Sulfonsäurealkyl-$C_1$-$C_4$-ester-, Sulfamoyl-, Alkyl-$C_1$-$C_4$-sulfon-, Hydroxy-alkylen-$C_1$-$C_4$-sulfon-, Phenylsulfon-, Hydroxyphenylsulfon-, Alkyl-$C_1$-$C_4$-phenylsulfon oder Alkoxy-$C_1$-$C_4$-phenylsulfongruppe bedeuten, erfolgte bisher in der Regel aus den zugrundeliegenden Nitroverbindungen der allgemeinen Formel (A) (siehe weiter unten), die zu den entsprechenden Aminoverbindungen der Formel (B) reduziert und anschließend mit einem Alkylierungsmittel der allgemeinen Formel R-$CH_2$-Z, in welcher R die vorstehend genannte Bedeutung hat und Z ein Chlor- oder Bromatom oder die Gruppierung -$OSO_2$-$OCH_2$-R (R hat hierin wiederum die vorstehend genannte Bedeutung), wie Alkylhalogenide oder Alkylsulfate, in Gegenwart stöchiometrischer Mengen eines Säurebinders zu den Zielverbindungen der genannten Formel (1) gemäß dem Reaktionsschema

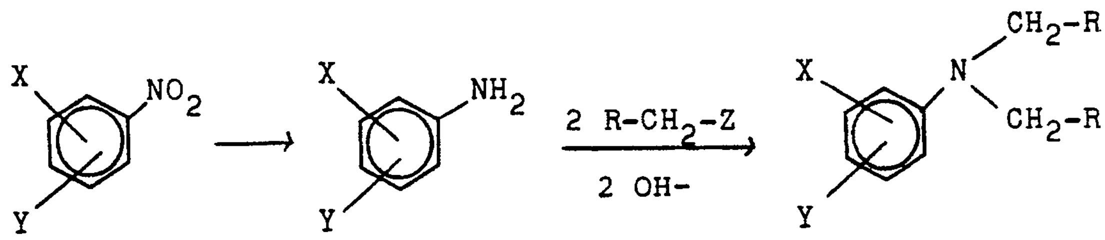


(A) (B) (1)

umgesetzt werden. Diese Verfahrensschritte sind jedoch aus gewerbehygienischer, ökologischer und wirtschaftlicher Sicht nicht optimal, weil

1) die Alkylierungsmittel der genannten allgemeinen Formel $R-CH_2-Z$ (eine der Ausgangsverbindungen) physiologisch bedenklich sind,

2) das Alkylierungsmittel im Überschuß angewendet werden muß,

3) Etherbildung als Nebenreaktion kaum vermeidbar ist,

4) eine hohe Salzbelastung der Abwässer mit Natriumchlorid/ Natriumsulfat und mit organischen Verbindungen (Alkanolen aus den Überschüssen (vgl. Punkt 2) gegeben ist,

5) Ausbeuteverluste durch Quaternierung (verbunden mit weiterer Abwasserbelastung) in Kauf zu nehmen sind,

6) meistens eine geringe Selektivität gegeben ist, und

7) das zu alkylierende Amin (B) (Ausgangsverbindung) in getrennter Reaktionsstufe aus der Nitroverbindung (A) hergestellt werden muß.

Es bestand daher ein Bedürfnis nach einem günstigeren Verfahren zur Herstellung aromatischer Dialkylamine, durch welches die genannten Nachteile vermieden werden. Bei der Suche nach einer geeigneten Lösung wurde die im Prinzip bekannte reduktive Alkylierung aromatischer Nitro- oder Aminoverbindungen mit Carbonylverbindungen näher in Betracht gezogen. Hierbei zeigte sich jedoch, daß nach dem Stand der Technik nur bei der Verwendung von Formaldehyd als Carbonylverbindung in breiterem Umfang eine Dialkylierung, zwangsläufig beschränkt auf eine Dimethylierung, möglich ist, wobei, abhängig von der zu alkylierenden Nitro- bzw. Aminoverbindung, wechselnde Ausbeuten erzielt werden [HOUBEN-WEYL 11/1, Seite 641 ff]. Hierbei können aber allenfalls unter technisch nur schwer zu realisierenden Bedingungen annehmbare Ergebnisse hinsichtlich Ausbeute und Produktqualität erzielt werden, wie durch reduktive Dimethylierung in korrosivem saurem Medium [JACS 73, 864 (1951); Belgische Patentschrift 832 296].

Die reduktive Dialkylierung aromatischer Nitro- oder Aminoverbindungen mit höheren Aldehyden der Formel

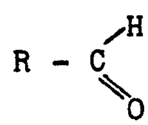

worin R die weiter oben angegebene Bedeutung hat, ist dagegen laut Literatur, soweit dort überhaupt erwähnt, nur unter technisch nicht praktikablen Bedingungen und mit unbefriedigenden Ausbeuten möglich. So werden bei der Umsetzung von Nitrobenzol mit aliphatischen Aldehyden der Formel

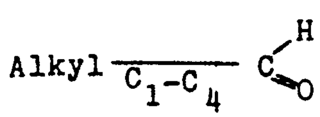

in Eisessig/Ethanol an $PtO_2$ bei Hydrierung unter Normaldruck die Dialkylaniline nach 96 Stunden in einer Ausbeute von 34-70 % erhalten [JACS 63, 749 (1941)].

Desgleichen gelingt die reduktive Diethylierung von 1-Amino-2-hydroxy-1,2-diphenylethan mit Acetaldehyd an $Pt/BaSO_4$ nur in Gegenwart von Aluminiumchlorid in 50%iger Ausbeute. Bei Abwesenheit von Aluminiumchlorid entsteht fast ausschließlich die Monoethylverbindung [Ber. 83, 66 (1950)].

Es wurde nun überraschenderweise gefunden, daß man entgegen dem durch die zitierten Literaturangaben verursachten Vorurteil aromatische Dialkylamine der weiter oben genannten allgemeinen Formel (1) in

hoher Ausbeute und Selektivität herstellen kann, indem man Verbindungen der allgemeinen Formel (2)

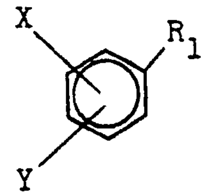

(2)

in welcher $R_1$ eine Nitro- oder primäre Aminogruppe, und X und Y die weiter oben genannten Bedeutungen haben, mit mindestens äquimolaren Mengen eines Aldehyds der allgemeinen Formel (3)

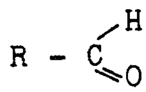

(3)

in welcher R die vorstehend genannte Bedeutung hat, in Alkoholen, wie Alkanolen($C_1$-$C_6$), beispielsweise Methanol, Ethanol, isomere Propanole, Butanole oder deren höhere Homologe; Alkylbenzolen, wie Toluol, Ethylbenzol oder isomere Xylole oder deren Gemische; Glykolethern, wie beispielsweise Methyl-, Ethyl- oder Butylglykol, Methyl-, Ethyl- oder Butyldiglykol, Methyl-, Ethyl- oder Butyltriglykoloder Methyl-, Ethyl- oder Butyltetraglykol; Fettsäure-dialkylamiden,wie beispielsweise Dimethylformamid; oder vorzugsweise in Fettsäure-alkylestern oder -glykolestern, wie beispielsweise Methyl-, Ethyl-, Butyl- oder Glykolester der Essig-, Propion- oder Buttersäure, und mit katalytisch aktiviertem Wasserstoff in Gegenwart eines Edelmetallkatalysators der 8. Gruppe des Periodischen Systems, beispielsweise Platin oder insbesondere Palladium, vorzugsweise auf geeignetem Trägermaterial (Kohle oder Bariumsulfat), ferner modifizierte Platinkatalysatoren, wie Platindioxid oder sulfidiertes oder sulfitiertes Platin, wobei der Katalysator zweckmäßigerweise in einer Menge von 1 bis 20 g/mol, vorzugsweise von 5 bis 10 g/mol, bezogen auf die Ausgangsverbindung der genannten Formel (2), eingesetzt wird, in Abwesenheit von Säure aber ggfs. in Gegenwart katalytischer Mengen eines Trialkyl-$C_1$-$C_6$-amins, wie beispielsweise Triethyl- oder Tributylamin in einer Menge von 1 bis 20 Mol-%, vorzugsweise 5 bis 10 Mol-%, bezogen auf die Ausgangsverbindung der genannten Formel (2) bei Temperaturen von 50° C bis 150° C, vorzugsweise 80° C bis 120° C, bei einem Druck von 20 bar bis 100 bar, vorzugsweise 40 bar bis 60 bar, reduktiv dialkyliert.

Während die Durchführung des erfindungsgemäßen Verfahrens in saurem Medium nachteilig ist, hat sich das Arbeiten in basischem Medium überraschenderweise häufig als vorteilhaft erwiesen. In der Literatur wurde bisher beim Arbeiten in basischem Medium nur eine selektive Monoalkylierung beschrieben (Organic Reactions 4, 174; JP-OS 57 123-148).

Bemerkenswerterweise gelingt unter den erfindungsgemäßen Bedingungen eine Dimethylierung der Ausgangsverbindungen der Formel (2) mittels Formaldehyd nicht, so daß der glatte Reaktionsablauf beim Einsatz der Aldehyde der genannten Formel (3) äußerst überraschend ist und in keiner Weise zu erwarten war. Das neue Verfahren liefert die aromatischen Dialkylamine (1) in ausgezeichneten Ausbeuten und sehr hoher Selektivität (< 1 % Monoalkylverbindung).

Da beim erfindungsgemäßen Verfahren der Zusatz von Säure, festen Hilfsstoffen, wie Aluminiumchlorid oder sauren Polymeren und Katalysator-Komplexen entfällt, kann es technisch optimal in jeder Hydrierapparatur, insbesondere den betrieblich eingeführten Hydrierern zur Produktion aromatischer Amine aus deren Nitrovorstufen durchgeführt werden. Hinzu kommt, daß sich die weiter oben aufgeführten Nachteile der bisherigen technischen Verfahren zur Herstellung aromatischer Dialkylamine vollständig vermeiden lassen und die Nitroverbindungen (Formel (2)) mit $R_1$ = Nitro sich in einer Eintopfreaktion direkt in die aromatischen Dialkylamine überführen lassen. Das erfindungsgemäße Verfahren stellt somit einen sehr erheblichen technischen Fortschritt dar.

Das Verfahren wird im einzelnen in der Weise durchgeführt, daß man Lösungen der Amine bzw. Nitroverbindungen (Formel (2)) mit ≧ 2 Mol eines Aldehyds der Formel (3) in den angegebenen Lösungsmitteln, ggf. in Gegenwart katalytischer Mengen tertiärer Amine, an den vorstehend genannten Katalysatoren bis zum Ende der Wasserstoffaufnahme hydriert.

Die Versuchsbedingungen sind hierbei wie folgt: Das Amin bzw. die Nitroverbindung der Formel (2) wird in den oben angegebenen Lösungsmitteln, vorzugsweise in n-Butylacetat, als 10 bis 50%ige Lösung (bei den Aminen haben sich 50%ige, bei den Nitroverbindungen 20%ige Lösungen als besonders vorteilhaft

erwiesen), ggf. in Anwesenheit katalytischer Mengen tertiärer Amine, insbesondere 10 Mol-% Triethylamin, bezogen auf umzusetzende Ausgangsverbindung, mit 2,0 bis 10,0 Mol, vorzugsweise 2,5 bis 3,5 Mol eines Aldehyds der Formel (3) bei 70° C bis 120° C, vorzugsweise bei 80 bis 100° C und 20 bis 100 bar, vorzugsweise 40 bis 60 bar Wasserstoffdruck an den obengenannten Edelmetallkatalysatoren, insbesondere an Palladium auf Kohle, 2 bis 8 Stunden hydriert. Nach Abtrennung des Katalysatorsund Abdestillieren des Lösungsmittels erhält man die Dialkylaminoverbindung der Formel (1) in hoher Ausbeute und Reinheit.
Die Identität der nach diesem Verfahren hergestellten Verbindungen ist durch $^{1}H$-NMR- und $^{13}C$-NMR-Spektren sichergestellt.

Beispiel 1

68,5 Teile p-Kresidin, 68,5 Teile Toluol und 5 Teile Katalysator (5 % Palladium/Kohle) werden im Autoklav vorgelegt. Der Autoklav wird verschlossen und mit Stickstoff gespült. Dann werden mittels einer Druckschleuse 132 Teile Acetaldehyd in einer Portion zugesetzt. Die reduktive Alkylierung erfolgt in 3 Stunden bei 100° C und 40 bis 60 bar Wasserstoffdruck. Anschließend wird der Katalysator abfiltriert und die leichtflüchtigen Bestandteile unter vermindertem Druck abdestilliert. Man erhält 99 Teile rohen N,N-Diethylamino-p-kresylmethylether (RG: 89,5 % (HPLC)), d.h. 92,7 % der Theorie.

Bei Austausch von Toluol gegen Xylol, Methylglykol, Ethylacetat oder Butanol erhält man vergleichbare Ergebnisse hinsichtlich Ausbeute und Reinheit.

Beispiel 2

Eine Lösung von 83,5 Teilen 3-Nitro-4-methoxytoluol in 352 Teilen n-Butylacetat wird im Autoklav vorgelegt und mit 5 Teilen Triethylamin sowie 5 Teilen Katalysator (5 % Palladium/Kohle) versetzt. Der Autoklav wird verschlossen und mit Stickstoff gespült. Dann werden mittels einer Druckschleuse 132 Teile Acetaldehyd in einer Portion zugesetzt. Die reduktive Alkylierung erfolgt in 2 Stunden bei 100° C und 40 bis 60 bar Wasserstoffdruck. Anschließend wird der Katalysator abgesaugt und die leichtflüchtigen Bestandteile unter vermindertem Druck abdestilliert. Man erhält 99 Teile rohen N,N-Diethylamino-p-kresylmethylether (RG: 90,8 % (HPLC)), d.h. 93,2 % der Theorie.

Beispiel 3

76,5 Teile Aminohydrochinondimethylether, 76,5 Teile Ethanol, 5 Teile Triethylamin und 5 Teile Katalysator (5 % Palladium/Kohle) werden im Autoklav vorgelegt. Der Autoklav wird verschlossen und mit Stickstoff gespült. Dann werden mittels einer Druckschleuse 110 Teile Acetaldehyd in einer Portion zugesetzt. Die reduktive Alkylierung erfolgt in 2 Stunden bei 100° C und 40 bis 60 bar Wasserstoffdruck. Anschließend wird der Katalysator abfiltriert und die leichtflüchtigen Bestandteile unter vermindertem Druck abdestilliert. Es verbleiben 105,6 Teile roher N,N-Diethylamino-hydrochinondimethylether (RG: 89,9 % (HPLC)), d.h. 90,8 % der Theorie.

Beispiel 4

Eine Lösung von 83,5 Teilen 4-Nitrobenzoesäure in 334 Teilen n-Butylacetat wird im Autoklav vorgelegt und mit 5 Teilen Triethylamin sowie 5 Teilen Katalysator (5 % Palladium/ Kohle) versetzt. Der Autoklav wird verschlossen und mit Stickstoff gespült. Dann werden mittels einer Druckschleuse 132 Teile Acetaldehyd in einer Portion zugesetzt. Die reduktive Alkylierung erfolgt in 2 Stunden bei 100° C und 40 bis 60 bar Wasserstoffdruck. Anschließend wird der Katalysator abfiltriert und die leichtflüchtigen Bestandteile unter vermindertem Druck abdestilliert. Man erhält 100 Teile roher 4-N,N-Diethyl-aminobenzoesäure (RG: 74,0 % (HPLC)), d.h. 76,7% der Theorie.

Ersetzt man das Butylacetat durch Glykoldiacetat und arbeitet im übrigen in der gleichen Weise, so erhält man das Zielprodukt in vergleichbarer Ausbeute und Qualität.

Beispiel 5

Eine Lösung von 52,5 Teilen 2-Nitro-4-acetaminoanisol in 210 Teilen n-Butylacetat wird im Autoklav vorgelegt und mit 2,5 Teilen Triethylamin sowie 2,5 Teilen Katalysator (5 % Palladium/Kohle) versetzt. Der Autoklav wird verschlossen und mit Stickstoff gespült. Dann werden mittels einer Druckschleuse 66 Teile Acetaldehyd in einer Portion zugesetzt. Die reduktive Alkylierung erfolgt in 2 Stunden bei 100° G und 40 bis

60 bar Wasserstoffdruck. Anschließend wird der Katalysator abfiltriert und die leichtflüchtigen Bestandteile unter vermindertem Druck abdestilliert. Es verbleiben 64,1 Teile rohes 2-Diethylamino-4-acetaminoanisol (RG: 88,5 % (HPLC)), d.h. 92,9 % der Theorie.

Beispiel 6

Eine Lösung von 18,3 Teilen Nitrohydrochinondimethylether in 164,7 Teilen Xylol wird im Autoklav vorgelegt und mit 58 Teilen Propionaldehyd sowie 1 Teil Katalysator (5 % Palladium/Kohle) versetzt. Der Autoklav wird verschlossen und mit Stickstoff gespült. Die reduktive Alkylierung erfolgt in 3 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erholt 25 Teile rohen N,N-Di-n-propylaminohydrochinondimethylether (RG: 91,0 % (HPLC)), d.h. 96,0 % der Theorie.

Ersetzt man den Palladium-Katalysator durch die gleiche Menge Platin/$BaSO_4$ und arbeitet im übrigen wie angegeben, so erhält man ein vergleichbares Ergebnis.

Beispiel 7

Eine Lösung von 76,5 Teilen m-Nitroanisol in 306 Teilen n-Butylacetat wird im Autoklav vorgelegt und mit 5 Teilen Triethylamin sowie 5 Teilen Katalysator (5 % Palladium/ Kohle) versetzt. Der Autoklav wird verschlossen und mit Stickstoff gespült. Dann werden mittels einer Druckschleuse 132 Teile Acetaldehyd in einer Portion zugesetzt. Die reduktive Alkylierung erfolgt in 3 Stunden bei 100 °C und 40 bis 60 bar Wasserstoffdruck. Anschließend wird der Katalysator abfiltriert und die leichtflüchtigen Bestandteile unter vermindertem Druck abdestilliert. Es verbleiben 89,1 Teile rohes N,N-Diethyl-m-anisidin (RG: 92,5 % (HPLC)), d.h. 78,1 % der Theorie.

Beispiel 8

89 Teile 3-Amino-butyranilid, 89 Teile n-Butylacetat, 5 Teile Triethylamin und 5 Teile Katalysator (5 % Palladium/Kohle) werden im Autoklav vorgelegt. Der Autoklav wird verschlossen und mit Stickstoff gespült. Dann werden mittels einer Druckschleuse 132 Teile Acetaldehyd in einer Portion zugesetzt. Die reduktive Alkylierung erfolgt in 2 h bei 70 bis 80 °C und 40 bis 60 bar Wasserstoffdruck. Anschließend wird der Katalysator abfiltriert und die leichtflüchtigen Bestandteile unter vermindertem Druck abdestilliert. Man erhält 115,4 Teile rohes 3-N,N-Diethylamino-butyranilid (RG: 91,2 % (HPLC)), d.h. 90 % der Theorie.

Beispiel 9

Eine Lösung von 18,3 Teilen Nitrohydrochinondimethylether in 164,7 Teilen n-Butylacetat wird im Autoklav vorgelegt und mit 72 Teilen n-Butyraldehyd sowie 1 Teil Katalysator (5 % Palladium/Kohle) versetzt. Der Autoklav wird verschlossen und mit Stickstoff gespült. Die reduktive Alkylierung erfolgt in 3 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erhält 28 Teile rohen N,N-Di-n-butylaminohydrochinondimethylether (RG: 86,1 % (HPLC)), d.h. 91,0 % der Theorie.

Beispiel 10

Eine Lösung von 91,5 Teilen Nitrohydrochinondimethylether in 366 Teilen n-Butylacetat wird im Autoklav vorgelegt und mit 5 Teilen Katalysator (5 % Palladium/Kohle) versetzt. Der Autoklav wird verschlossen und mit Stickstoff gespült. Dann werden mittels einer Druckschleuse 154 Teile Acetaldehyd in einer Portion zugesetzt. Die reduktive Alkylierung erfolgt in 3 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erhält 105,8 Teile rohen N,N-Diethylamino-hydrochinondimethylether (RG: 96,0 % (HPLC)), d.h. 97,2 % der Theorie.

Beispiel 11

Eine Lösung von 90 Teilen 3-Nitroacetanilid in 270 Teilen n-Butylacetat wird im Autoklav vorgelegt und mit 5 Teilen Katalysator (5 % Palladium/Kohle) versetzt. Der Autoklav wird verschlossen und mit Stickstoff gespült. Dann werden mittels einer Druckschleuse 154 Teile Acetaldehyd in einer Portion zugesetzt. Die

reduktive Alkylierung erfolgt in 3 Stunden bei 100 °C und 40 bis 60 bar Wasserstoffdruck. Anschließend wird der Katalysator abgesaugt und die leichtflüchtigen Bestandteile unter vermindertem Druck abdestilliert. Es verbleiben 107,4 Teile rohes 3-N,N-Diethylaminoacetanilid (RG: 90,5 % (HPLC)), d.h. 94,4 % der Theorie.

Beispiel 12

Eine Lösung von 13,9 Teilen o-Nitrophenol in 125 Teilen n-Butylacetat wird im Autoklav vorgelegt und mit 1 Teil Katalysator (5 % Palladium/Kohle) versetzt. Der Autoklav wird verschlossen und mit Stickstoff gespült. Dann werden mittels einer Druckschleuse 44 Teile Acetaldehyd in einer Portion zugesetzt. Die reduktive Alkylierung erfolgt in 4 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erhält 17,5 Teile rohes 2-N,N-Diethylaminophenol (RG: 78,8 % (HPLC)), d.h. 88,4 % der Theorie.

Beispiel 13

Eine Lösung von 57,8 Teilen 3-Nitrophenyl-β-oxethylsulfon in 115 Teilen n-Butylacetat wird im Autoklav vorgelegt und mit 2,5 Teilen Katalysator (5 % Palladium/Kohle) versetzt. Der Autoklav wird verschlossen und mit Stickstoff gespült. Dann werden mittels einer Druckschleuse 77 Teile Acetaldehyd in einer Portion zugesetzt. Die reduktive Alkylierung erfolgt in 4 Stunden bei 100 °C und 40 bis 60 bar Wasserstoffdruck. Anschließend wird der Katalysator abfiltriert und die flüchtigen Bestandteile unter vermindertem Druck abdestilliert. Man erhält 59,8 Teile rohes 3-N,N-Diethylaminophenyl-β-oxethylsulfon (RG: 97,0 % (HPLC)), d.h. 90,3 % der Theorie.

Beispiel 14

Eine Lösung von 75,5 Teilen 4-Nitroethylbenzol in 302 Teilen Xylol wird im Autoklav vorgelegt und mit 5 Teilen Katalysator (5 % Palladium/Kohle) sowie 116 Teilen Propionaldehyd versetzt. Der Autoklav wird verschlossen und mit Stickstoff gespült. Die reduktive Alkylierung erfolgt in 4 Stunden bei 100 °C und 40 bis 60 bar Wasserstoffdruck. Anschließend wird der Katalysator abfiltriert und die leicht flüchtigen Bestandteile unter vermindertem Druck abdestilliert. Man erhält 105 Teile rohes 4-N,N-Di-n-propylaminoethylbenzol (RG: 92,7 % (HPLC)), d.h. 95 % der Theorie.

Beispiel 15

Eine Lösung von 90,5 Teilen 3-Nitrophenyl-n-propylether in 362 Teilen n-Butylacetat wird im Autoklav vorgelegt und mit 5 Teilen Katalysator (5 % Palladium/Kohle) versetzt. Der Autoklav wird verschlossen und mit Stickstoff gespült. Dann werden mittels einer Druckschleuse 132 Teile Acetaldehyd in einer Portion zugesetzt. Die reduktive Alkylierung erfolgt in 4 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erhält 108 Teile rohen 3-N,N-Diethylaminophenyl-n-propylether (RG: 89,5 % (HPLC)), d.h. 93,4 % der Theorie.

Ersetzt man den Palladium-Katalysator durch gleiche Mengen $PtO_2$/Kohle und arbeitet im übrigen in der gleichen Weise, so erhält man ein vergleichbares Ergebnis.

Beispiel 16

Eine Lösung von 24,6 Teilen Nitrobenzol in 98,4 Teilen n-Butylacetat wird im Autoklav vorgelegt und mit 100,8 Teilen n-Butyraldehyd sowie 2 Teilen Katalysator (5 % Palladium/Kohle)versetzt. Der Autoklav wird verschlossen und mit Stickstoff gespült. Die reduktive Alkylierung erfolgt in 3 Stunden bei 100 °C und 40 bis 60 bar Wasserstoffdruck. Anschließend wird der Katalysator abgesaugt und die leichtflüchtigen Bestandteile unter vermindertem Druck abdestilliert. Man erhält 43 Teile rohes N,N-Di-n-butylanilin (RG: 85,2 % (HPLC)), d.h. 89,7 % der Theorie.

Beispiel 17

Eine Lösung von 104 Teilen 4-Nitrobenzoesäure-n-propylester in 418 Teilen n-Butylacetat wird im Autoklav vorgelegt und mit 174 Teilen Propionaldehyd sowie 5 Teilen Katalysator (5 % Palladium/Kohle)

versetzt. Der Autoklav wird verschlossen und mit Stickstoff gespült. Die reduktive Alkylierung erfolgt in 3 Stunden bei 100 °C und 40 bis 60 bar Wasserstoffdruck. Anschließend wird der Katalysator abfiltriert und die leichtflüssigen Bestandteile unter vermindertem Druck abdestilliert. Man erhält 135 Teile rohen 4-N,N-Di-n-propylaminobenzoesäure-n-propylester (RG: 84,3 % (HPLC)), d.h. 86,5 % der Theorie.

Beispiel 18

Eine Lösung von 90,5 Teilen 3-Nitro-4-ethoxytoluol in 362 Teilen n-Butylacetat wird im Autoklav vorgelegt und mit 5 Teilen Katalysator (5 % Palladium/Kohle) vesetzt. Der Autoklav wird verschlossen und mit Stickstoff gespült. Dann werden mittels einer Druckschleuse 88 Teile Acetaldehyd in einer Portion zugesetzt. Die reduktive Alkylierung erfolgt in 2 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erhält 104 Teile rohen N,N-Diethylamino-p-kresylethylether (RG: 98,5 % (HPLC)), d.h. 90,0 % der Theorie.

Beispiel 19

Eine Lösung von 47,2 Teilen m-Nitrochlorbenzol in 425 Teilen n-Butylacetat wird im Autoklav vorgelegt und mit 4,5 Teilen Katalysator (5 % Platin/S/Kohle) versetzt. Der Autoklav wird verschlossen und mit Stickstoff gespült. Dann werden mittels einer Druckschleuse 44 Teile Acetaldehyd in einer Portion zugesetzt. Die reduktive Alkylierung erfolgt in 5 Stunden bei 100 °C und 40 bis 60 bar Wasserstoffdruck. Anschließend wird der Katalysator abfiltriert und die leichtflüchtigen Bestandteile unter vermindertem Druck abdestilliert. Man erhält 53 Teile rohes 3-Chlor-N,N-diethylanilin (RG: 92,5 % (HPLC)), d.h. 89,1 % der Theorie.

Beispiel 20

Eine Lösung von 40,4 Teilen 3-Nitrobenzolsulfonamid in 80,8 Teilen Dimethylformamid wird im Autoklav vorgelegt und mit 4 Teilen Katalysator (5 % Palladium/Kohle) versetzt. Der Autoklav wird verschlossen und mit Stickstoff gespült. Dann werden mittels einer Druckschleuse 88 Teile Acetaldehyd in einer Portion zugesetzt. Die reduktive Alkylierung erfolgt in 6 Stunden bei 140 °C und 40 bis 60 bar Wasserstoffdruck. Nach Abfiltrieren des Katalysators werden die leichtflüchtigen Bestandteile unter vermindertem Druck abdestilliert. Man erhält 44 Teile rohes N,N-Diethylaminobenzolsulfonamid (RG: 88 % (HPLC)), d.h. 84,8 % der Theorie.

**Ansprüche**

**1.** Verfahren zur Herstellung von aromatischen Dialkylaminen der allgemeinen Formel (1)

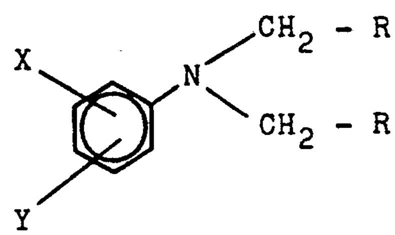

(1)

in welcher R eine Alkyl-$C_1$-$C_6$-Gruppe, X und Y je ein Wasserstoff-, Fluor- oder Chloratom oder eine Hydroxy-, Alkyl-$C_1$-$C_4$-, Alkoxy-$C_1$-$C_4$-, Carboxy-, Carbalkoxy-$C_1$-$C_5$-, Alkyl-$C_1$-$C_4$-CO-NH-,

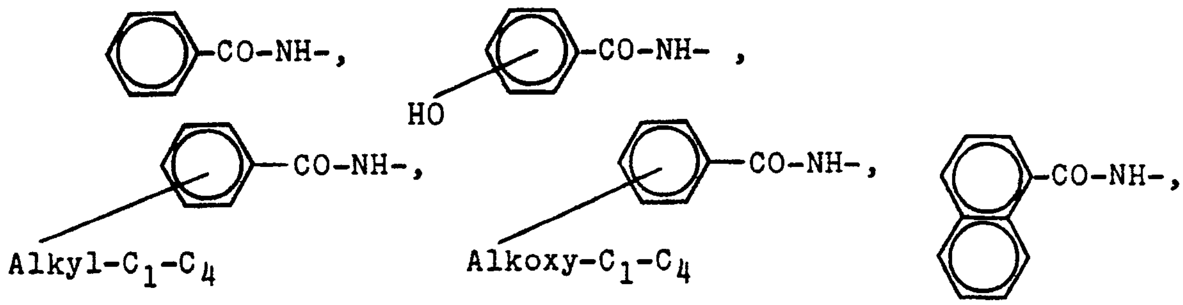

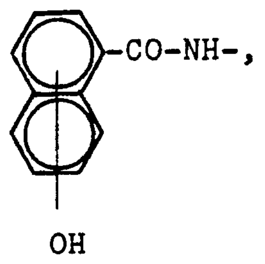 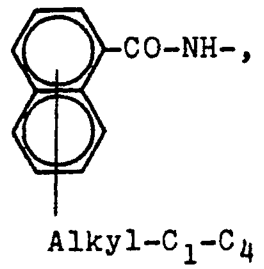 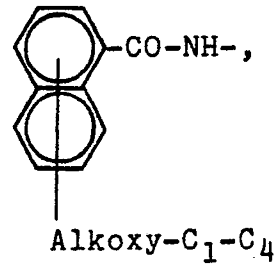

Sulfonsäure-, Sulfonsäurealkyl-$C_1$-$C_4$-ester-, Sulfamoyl-, Alkyl-$C_1$-$C_4$-sulfon-, Hydroxy-alkylen-$C_1$-$C_4$-sulfon-, Phenylsulfon-, Hydroxyphenylsulfon-, Alkyl-$C_1$-$C_4$-phenylsulfon oder Alkoxy-$C_1$-$C_4$-phenylsulfongruppen bedeuten, dadurch gekennzeichnet, daß man Verbindungen der allgemeinen Formel (2)

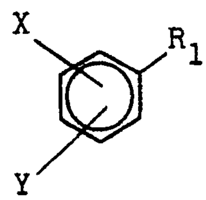 (2)

in welcher $R_1$ eine Nitro- oder primäre Aminogruppe, und X und Y die weiter oben genannten Bedeutungen haben, mit mindestens äquimolaren Mengen eines Aldehyds der allgemeinen Formel (3)

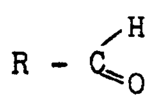 (3)

in welcher R die vorstehend genannte Bedeutung hat, in Alkoholen, Alkylbenzolen, Glykolethern, Fettsäuredialkylamiden oder Fettsäurealkylestern oder -glykolestern, und mit katalytisch aktiviertem Wasserstoff in Gegenwart eines Edelmetallkatalysators der 8. Gruppe des periodischen Systems, in Abwesenheit von Säure aber ggfs. in Gegenwart katalytischer Mengen eines Trialkyl-$C_1$-$C_6$-amins, bei Temperaturen von 50° C bis 150° C bei einem Druck von 20 bar bis 100 bar reduktiv dialkyliert.

**2.** Verfahren nach Anspruch 1, dadurch gekennzeichnet, daß man die reduktive Dialkylierung in einem Alkanol von 1 bis 6 Kohlenstoffatomen, Toluol, Ethylbenzol, einem isomeren Xylol oder einem Gemisch aus diesen aromatischen Kohlenwasserstoffen, Methyl-, Ethyl- oder Butyldiglykol, Methyl-, Ethyl- oder Butyltriglykol, Methyl-, Ethyl- oder Butyltetraglykol, Methyl-, Ethyl-, Butyl- oder Glykolester der Essig-, Propion- oder Buttersäure oder in Dimethylformamid durchführt.

**3.** Verfahren nach Anspruch 1, dadurch gekennzeichnet, daß man die reduktive Dialkylierung in Gegenwart von Platin, Palladium, Platindioxid oder sulfidiertem oder sulfitiertem Platin als Katalysatoren durchführt.

**4.** Verfahren nach Anspruch 1, dadurch gekennzeichnet, daß man die reduktive Dialkylierung in Gegenwart katalytischer Mengen von Triethylamin oder Tributylamin durchführt.

5. Verfahren nach Anspruch 1, dadurch gekennzeichnet, daß man die reduktive Dialkylierung bei Temperaturen von 80° C bis 120° C und bei einem Druck von 40 bar bis 60 bar durchführt.

## Claims

1. A process for the preparation of aromatic dialkylamines of the formula (1)

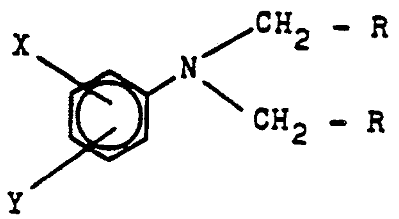

(1)

in which R denotes an alkyl-$C_1$-$C_6$ group, X and Y each denote a hydrogen, fluorine or chlorine atom or a hydroxyl, alkyl-$C_1$-$C_4$, alkoxy-$C_1$-$C_4$, carboxyl, carbalkoxy-$C_1$-$C_5$, alkyl-$C_1$-$C_4$-CO-NH-,

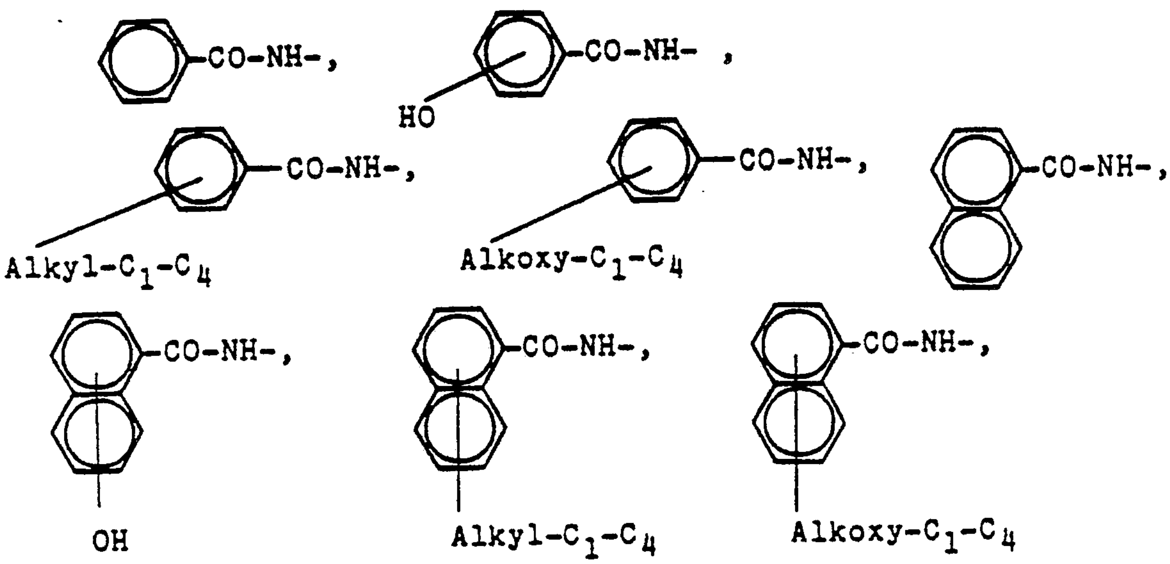

sulfonic acid, sulfonicacidalkyl-$C_1$-$C_4$ ester, sulfamoyl, alkyl-$C_1$-$C_4$-sulfonyl, hydroxy-alkylene-$C_1$-$C_4$-sulfonyl, phenylsulfonyl, hydroxyphenylsulfonyl, alkyl-$C_1$-$C_4$-phenylsulfonyl or alkoxy-$C_1$-$C_4$-phenylsulfonyl group, which comprises reductively dialkylating compounds of the formula (2)

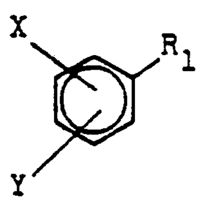

(2)

in which $R_1$ denotes a nitro or primary amino group, and X and Y have the abovementioned meanings, using at least equimolar amounts of an aldehyde of the formula (3)

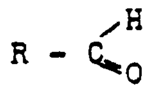

(3)

in which R has the abovementioned meaning, in alcohols, alkylbenzenes, glycol ethers, fatty acid dialkylamides or fatty acid alkyl esters or fatty acid glycol esters, and using catalytically activated hydrogen in the presence of a precious metal catalyst from group 8 of the periodic table, in the absence of acid, but if appropriate in the presence of catalytic amounts of a trialkyl-$C_1$-$C_6$-amine, at temperatures from 50 °C to 150 °C at a pressure from 20 bar to 100 bar.

2. The process as claimed in claim 1, wherein the reductive dialkylation is carried out in an alkanol having 1 to 6 carbon atoms, toluene, ethyl benzene, an isomeric xylene or a mixture of these aromatic hydrocarbons, methyl, ethyl or butyl diglycol, methyl, ethyl or butyl triglycol, methyl, ethyl or butyl tetraglycol, methyl, ethyl, butyl or glycol esters of acetic acid, propionic acid or butyric acid, or in dimethyl formamide.

3. The process as claimed in claim 1, wherein the reductive dialkylation is carried out in the presence of platinum, palladium, platinum dioxide or sulfided or sulfited platinum as catalysts.

4. The process as claimed in claim 1, wherein the reductive dialkylation is carried out in the presence of catalytic amounts of triethylamine or tributylamine.

5. The process as claimed in claim 1, wherein the reductive dialkylation is carried out at temperatures from 80 °C to 120 °C and at a pressure of 40 bar to 60 bar.

**Revendications**

1. Procédé pour préparer des dialkylamines aromatiques répondant à la formule générale 1 :

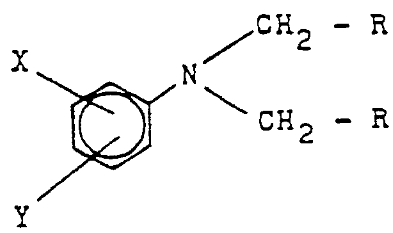

(1)

dans laquelle R représente un alkyle contenant de 1 à 6 atomes de carbone, et X et Y représentent chacun un atome d'hydrogène, un atome de fluor ou de chlore, un hydroxy, un alkyle en $C_1$-$C_4$, un alcoxy en $C_1$-$C_4$, un carboxy, un alcoxycarbonyle à alkyle en $C_1$-$C_5$, un alkyl-carbonylamino à alkyle en $C_1$-$C_4$, un radical répondant à l'une des formules :

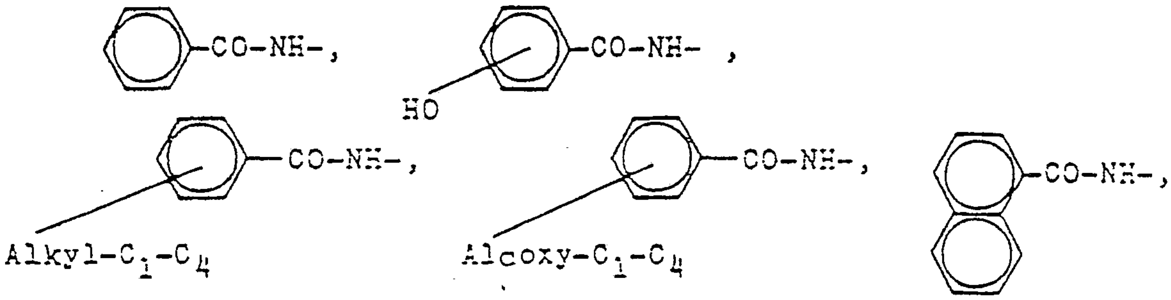

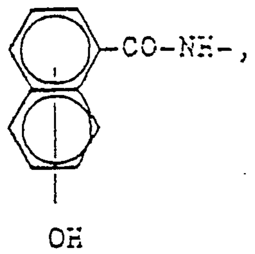

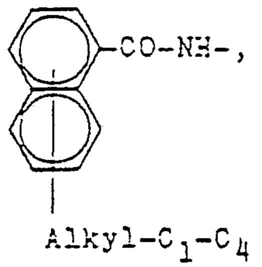

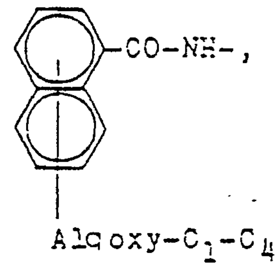

un sulfo, un alcoxysulfonyle en $C_1$-$C_4$, un sulfamoyle, un alkylsulfonyle en $C_1$-$C_4$, un hydroxy-alkylène-sulfonyle en $C_1$-$C_4$, un phénylsulfonyle, un hydroxyphényl-sulfonyle, un (alkyl-$C_1$-$C_4$)-phénylsulfonyle ou un (alcoxy-$C_1$-$C_4$)-phénylsulfonyle, procédé caractérisé en ce qu'on dialkyle de façon réductrice des composés répondant à la formule générale 2 :

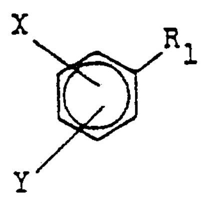

(2)

dans laquelle $R_1$ représente un radical nitro ou un radical amino primaire, et X et Y ont les significations qui leur ont été données ci-dessus, avec une quantité au moins équimolaire d'un aldéhyde répondant à la formule générale 3 :

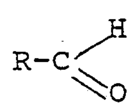

(3)

dans laquelle R a la signification qui lui a été donnée ci-dessus, dans des alcools, des alkylbenzènes, des éthers de glycols, des alkylamides d'acides gras, des esters alkyliques d'acides gras ou des esters dérivant de glycols et d'acides gras, et avec de l'hydrogène catalytiquement activé, en présence d'un catalyseur à base d'un métal noble du huitième groupe de la classification périodique, en l'absence d'acide mais, éventuellement, en présence de quantités catalytiques d'une tri-(alkyl-$C_1$-$C_6$)-amine, à des températures de 50 à 150 °C et sous une pression de 20 à 100 bar.

2. Procédé selon la revendication 1 caractérisé en ce que la dialkylation réductrice est effectuée dans un alcanol contenant de 1 à 6 atomes de carbone, dans le toluène, dans l'éthylbenzène, dans un xylène isomère ou dans un mélange de ces hydrocarbures aromatiques, dans le méthyl-, l'éthyl- ou le butyl-diglycol, dans le méthyléthyl-ou butyl-triglycol, dans le méthyl-, éthyl- ou butyl-tétraglycol, dans l'ester méthylique, éthylique, butylique ou glycolique de l'acide acétique, propionique ou butyrique, ou dans le diméthylformamide.

3. Procédé selon la revendication 1 caractérisé en ce qu'on effectue la dialkylation réductrice en présence de platine, de palladium, de dioxyde de platine ou de platine sulfuré ou sulfité comme catalyseur.

4. Procédé selon la revendication 1 caractérisé en ce qu'on effectue la dialkylation réductrice en présence de quantités catalytiques de triéthylamine ou de tributylamine.

5. Procédé selon la revendication 1 caractérisé en ce qu'on effectue la dialkylation réductrice à des températures de 80 à 120° C et sous une pression de 40 à 60 bar.